# EUROPEAN PATENT APPLICATION

(11) **EP 4 484 363 A1**
(43) Date of publication of application: **01.01.2025**
(21) Application number: 23182233.9
(22) Date of filing: 28.06.2023
(51) Int. Cl.: C01B 3/32, C07C 29/151, C07C 31/04, C10G 2/00, C25B 1/04

(54) **INTEGRATED PROCESSES FOR UTILIZING SYNTHESIZED AND STORED METHANOL**

(71) Applicant: BP P.L.C., London SW1Y 4PD (GB)
(72) Inventor: The designation of the inventor has not yet been filed
(74) Representative: Hamer, Christopher K.

(57) **Abstract**

The present disclosure relates generally to integrated processes for the production, storage, and use of methanol. In one aspect, the present disclosure provides a process for producing a H₂/CO stream, the process comprising for a first period of time, synthesizing methanol by hydrogenation of CO₂, and decomposing a second feed stream including the methanol to form CO and Hz; and for a second period of time, decomposing a third feed stream comprising stored methanol to form CO and H₂. A synthesized methanol fraction of the second feed stream is substantially greater than a synthesized methanol fraction of the third feed stream.

## Description

### BACKGROUND OF THE DISCLOSURE

### FIELD

The present disclosure relates to integrated processes for the utilization of synthesized and stored methane, such as in the production of syngas.

### TECHNICAL BACKGROUND

The conversion of synthesis gas into hydrocarbons by the Fischer-Tropsch process has been known for many years. The growing importance of alternative energy sources has resulted in renewed interest in the Fischer-Tropsch (FT) process as it allows an alternative route to high-quality fuels and feedstock chemicals through use of bio-derived carbon sources.

FT processes are typically used to produce linear hydrocarbons, which may be used in the production of fuels, as well as oxygenates which can also be useful in the production of fuels and otherwise serve as valuable feedstock chemicals.

A variety of transition metals have been identified to be catalytically active in the conversion of synthesis gas into hydrocarbons and oxygenated derivatives thereof. In particular, cobalt, nickel, and iron have been studied, often in combination with a support material, of which the most common are alumina, silica and carbon.

Synthesis gas (syngas) is comprised of carbon monoxide and hydrogen. While hydrogen is conventionally produced from natural gas or other fossil fuel sources, it can be also be produced through water electrolysis using electricity produced from a renewable source of energy, leading to a hydrogen product with potentially lower associated carbon dioxide emissions. However, renewable sources of energy, such as wind or solar, suffer from significant variability which makes their incorporation into steady-state processes, such as Fischer-Tropsch synthesis, challenging.

Accordingly, there remains a need to develop processes to more efficiently utilize renewable sources of energy in the production of syngas.

### SUMMARY

The present inventors have developed integrated processes for the production and storage of methanol, which can then be decomposed to generated syngas. Advantageously, these processes can incorporate hydrogen generated using a renewable source of energy and can serve to smooth the intermittent energy availability sometimes associated with the use of renewable sources, allowing for increased consistency of methanol supply and thus increased consistency of syngas production.

Thus, in one aspect, the disclosure provides for processes for production of a H₂/CO stream comprising hydrogen and carbon monoxide, the process comprising:
for a first period of time,
   providing a first feed stream comprising hydrogen and CO₂;
   contacting the first feed stream with a hydrogenation catalyst (e.g., in a hydrogenation reactor) to form a first product stream comprising synthesized methanol;
   providing a second feed stream comprising at least a portion of the synthesized methanol of the first product stream;
   contacting the second feed stream with a methanol decomposition catalyst (e.g., in a methanol decomposition reactor) to decompose at least a portion of the methanol to form a second product stream comprising CO and H₂; and
   providing at least a portion of the CO and the H₂ of the second product stream to the H₂/CO stream; and
for a second period of time,
   providing a source of stored methanol;
   providing a third feed stream comprising stored methanol;
   contacting the third feed stream with the methanol decomposition catalyst (e.g., in a methanol decomposition reactor) to decompose at least a portion of the methanol to form a third product stream comprising CO and H₂; and
   providing at least a portion of the CO and the H2 of the third product stream to the H₂/CO stream,
wherein a synthesized methanol fraction of the second feed stream is substantially greater than a synthesized methanol fraction of the third feed stream.

In another aspect, the present disclosure provides for processes for the production of hydrocarbons, the process comprising performing a Fischer-Tropsch reaction by contacting at least a portion of the H₂/CO stream as otherwise described herein with a Fischer-Tropsch catalyst (e.g., in a Fischer-Tropsch reactor) to provide a Fischer-Tropsch product stream comprising hydrocarbons, water, and optionally oxygenated hydrocarbons.

Other aspects of the disclosure will be apparent to those skilled in the art in view of the description that follows.

### BRIEF DESCRIPTION OF THE DRAWINGS

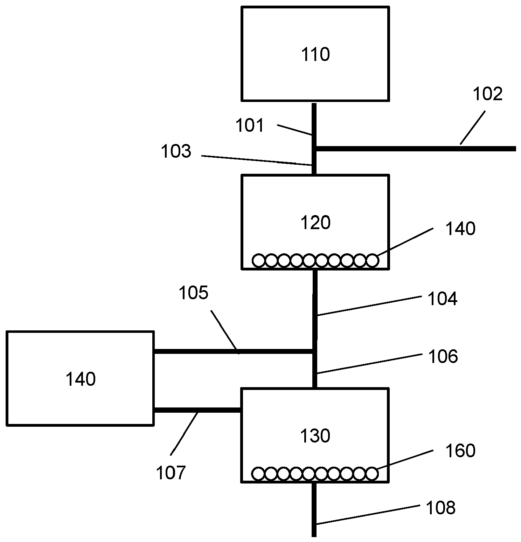

Fig. 1 provides a process schematic according to one embodiment of the disclosure.

### DEAILED DESCRIPTION

Energy derived from renewable sources of often suffers from intermittency in availability, with large increases and decreases associated with the availability of the source. For example, solar energy can only produce usable energy during daytime, and wind energy is only effective during windy periods. However, many industrial processes require steady-state operation in order to run efficiently, and frequent process interruptions can lead to increased energy usage, diminished product output and/or quality, and/or increased wear on the process machinery, potentially leading to lower lifetimes of capital equipment. Furthermore, effective large-scale energy storage methods are often prohibitively expensive or inefficient. The present disclosure provides methods for managing energy-associated intermittency, for example the intermittency of hydrogen produced through the use of electrolysis powered by renewable sources of energy. Advantageously, the present inventors have recognized that methanol can serve as a storage vehicle for syngas, as it can be easily transported and stored as a liquid. Thus, stored methanol can serve as a buffer against intermittent syngas production.

Thus, in one aspect, the disclosure provides for processes for production of a H₂/CO stream comprising hydrogen and carbon monoxide, the process comprising:
for a first period of time,
   providing a first feed stream comprising hydrogen and CO₂;
   contacting the first feed stream with a hydrogenation catalyst (e.g., in a hydrogenation reactor) to form a first product stream comprising synthesized methanol;
   providing a second feed stream comprising at least a portion of the synthesized methanol of the first product stream;
   contacting the second feed stream with a methanol decomposition catalyst (e.g., in a methanol decomposition reactor) to decompose at least a portion of the methanol to form a second product stream comprising CO and H₂; and
   providing at least a portion of the CO and the H₂ of the second product stream to the H₂/CO stream; and
for a second period of time,
   providing a source of stored methanol;
   providing a third feed stream comprising stored methanol;
   contacting the third feed stream with the methanol decomposition catalyst (e.g., in a methanol decomposition reactor) to decompose at least a portion of the methanol to form a third product stream comprising CO and H₂; and
   providing at least a portion of the CO and the H₂ of the third product stream to the H₂/CO stream,
wherein a synthesized methanol fraction of the second feed stream is substantially greater than a synthesized methanol fraction of the third feed stream.

As used herein, the term "feed stream" is used to mean the total material input to a process step, e.g., CO₂ hydrogenation, methanol decomposition, or Fischer-Tropsch reaction, regardless of whether provided in a single physical stream or multiple physical streams, and whether through a single inlet or multiple inlets. For example, H₂ and CO₂ of the first feed stream can be provided to a hydrogenation reactor in a single physical stream (e.g., in a single pipe to the reactor), or in multiple physical streams (e.g., separate inlets for CO₂ and H₂, or one inlet for fresh CO₂ and H₂ and another for recycled CO₂ and/or H₂). Similarly, a "product stream" is used to refer to the total material output from a process step, e.g., hydrogenation of carbon dioxide, methanol decomposition, or Fischer-Tropsch reaction, regardless of whether provided in a single physical stream or multiple physical streams, and whether through a single reactor outlet or multiple reactor outlets.

Hydrogen may be categorized based on the process used to produce it. Accordingly, in various embodiments as otherwise described herein, at least a portion of the hydrogen (e.g., at least 50%, at least 75%, at least 90% or at least 95%) of the first feed stream is one or more of grey hydrogen (produced conventionally from natural gas, or methane using steam reformation without capturing associated greenhouse gases), brown or black hydrogen (hydrogen produced using black coal or lignite), green hydrogen (made using water electrolysis powered by electricity from renewable sources of energy), pink hydrogen (made from electrolysis powered by nuclear energy), turquoise hydrogen (made from methane pyrolysis to produce hydrogen and solid carbon), blue hydrogen (made from steam reforming of natural gas, where the resulting carbon dioxide is captured), yellow hydrogen (made from electrolysis using solar power) and orange hydrogen (made from electrolysis using wind power). In particular embodiments, at least a portion of the hydrogen of the first feed stream is one or more of green hydrogen, pink hydrogen, or blue hydrogen (e.g., green hydrogen or blue hydrogen). The person of ordinary skill in the art is familiar with the color designations of various types of hydrogen. Biomass gasification may also be used to generate hydrogen.

Advantageously, the process as otherwise described herein may incorporate hydrogen gas prepared through electrolysis. Thus, in various embodiments, the process further comprises electrolyzing water to form an electrolysis product stream comprising hydrogen, and providing hydrogen from the electrolysis product stream to the first feed stream. In various embodiments as otherwise described herein, the hydrogen of the first feed stream comprises green hydrogen. For example, in particular embodiments, the process further comprises electrolyzing water using electricity from a renewable source to form an electrolysis product stream comprising hydrogen. Electrolysis is an attractive source of hydrogen gas as it may be principally powered by renewable sources of energy. Accordingly, in various embodiments, the electrolyzing as otherwise described herein is performed using renewable sources of electricity. Examples of renewable sources of electricity include solar power (including photovoltaic solar, and solar thermal power), hydroelectric power (e.g., tidal energy), wind power, and geothermal power. Other examples include power derived from biomass. Numerous methods of electrolysis are known in the art. For example, electrolysis may be performed with water to produce a hydrogen gas containing stream, In particular embodiments, the hydrogen is formed through the electrolysis of a water solution (e.g., pure water). Water electrolysis is described further in U.S. Patent No. 4,312,720, U.S. Patent No. 4,021,323, and U.S. Patent No. 4,094,751, each of which is incorporated by reference in their entirety.

In various embodiments as described herein, methanol synthesis through hydrogenation may be performed by the reaction of hydrogen with carbon dioxide. In particular, carbon dioxide is a common waste gas that can act as a greenhouse gas when released into the atmosphere. In various particular embodiments, the first feed stream comprises no more than 10 vol% CO (e.g., no more than 5 vol%, or 3 vol%, or 2 vol%, or 1 vol%, or 0.1 vol% CO). In some embodiments, the first feed stream comprises substantially no CO.

Optionally, the first feed stream may also comprise one or more additional gases. In particular embodiments, the first feed stream comprises an inert carrier gas, wherein the inert carrier gas includes one or more of CH₄ or N₂, e.g., N₂. In various embodiments as otherwise described herein, at least 50 vol% of the of the first feed stream is made up of the combination of hydrogen, carbon dioxide and nitrogen, e.g., at least 60 vol%, at least 70 vol%, at least 80 vol%, or at least 90 vol%.

Carbon dioxide is a common waste material, and often desirable to be removed from waste streams rather than be vented to the atmosphere. Such capture of carbon dioxide is critical to the implementation of many initiatives as it may be one pathway to lower the carbon emissions of the associated process. Advantageously, the carbon dioxide utilized in the processes described herein may be carbon dioxide collected from the atmosphere or that would otherwise have been released into the atmosphere, e.g., from a combustion or other industrial process. The carbon dioxide may be captured, where it is collected or absorbed after release from an industrial process, or harvested directly from the atmosphere. Methods of carbon dioxide capture are known to those of skill in the art. In various embodiments, at least a portion of the CO₂ of the first feed stream is from direct air capture. Additionally or alternatively, carbon dioxide is often scrubbed from industrial effluent, especially processes that generate large amounts of carbon dioxide as a byproduct.

In various embodiments as otherwise described herein, the carbon dioxide of the first feed stream comprises captured carbon dioxide and/or carbon dioxide from biomass gasification, e.g., comprises at least 50%, at least 75%, at least 90% or at least 95% captured carbon dioxide and/or carbon dioxide from biomass gasification.

One source of biomass is agricultural products in the form of dedicated energy crops such as switchgrass, miscanthus, bamboo, sorghum, tall fescue, kochia, wheatgrass, poplar, willow, silver maple, eastern cottonwood, green ash, black walnut, sweetgum, and sycamore. Another biomass source is agricultural waste or agricultural crop residue. Conventional agricultural activities, including the production of food, feed, fiber, and forest products, generate large amounts of waste plant material. Examples of such materials include corn stover, wheat straw, oat straw, barley straw, sorghum stubble, and rice straw. A third biomass source is through forestry residues left after timber operations. Biomass may also be in the form of commercial waste, industrial waste, sewage sludge, and municipal waste, which includes commercial and residential garbage, including yard trimmings, paper and paperboard, plastics, rubber, leather, textiles, and food waste. Accordingly, in various embodiments as otherwise described herein, at least a portion (e.g., at least 20%, at least 50%, at least 75%, at least 90% or at least 95%) of the CO₂ of the first feed stream is derived from biomass gasification, for example, agricultural biomass or municipal waste biomass. Additional sources of agricultural biomass will be apparent to one of skill in the art as dictated by local availability, economics, and process compatibility.

To generate carbon dioxide from a carbon-containing material, such as biomass, the material is typically subjected to gasification. Gasification involves heating the material under controlled conditions to generate gaseous streams of carbon monoxide, hydrogen, and carbon dioxide. Controlled amounts of other reactants, such as oxygen and/or steam, may be used to tune the process. Gasification conditions are tuned in accordance with the carbon-containing material being gasified in order to efficiently produce gaseous products. The biomass may be any source as described above, or from multiple sources may be combined.

Biomass gasification can, in some embodiments, produce gas mixtures comprises carbon dioxide in admixture with other gases, such as methane and/or hydrogen sulfide. Accordingly, in various embodiments, the first feed stream comprises carbon dioxide derived from biogas. For example, entrained methane may also be included. Thus, in various embodiments, the first feed stream further comprises methane. However, in some embodiments relatively low levels of methane are desirable, for example, no more than 50 mol% of carbon-bearing species, e.g., no more than 30 mol%, or no more than 20 mol%, or no more than 10 mol%, or no more than 5 mol%. Hydrogen sulfide is a common component of biogas. However, in various embodiments, hydrogen sulfide may have a deleterious effect on certain catalysts of the process as disclosed herein. Accordingly, in various embodiments as otherwise described herein, the biogas further comprises a scrubbing step to remove at least a portion of the hydrogen sulfide.

Carbon from renewable sources, such as biomass gasification, may be differentiated from fossil-derived carbon through techniques known in the art, for example through carbon radioisotope analysis.

In various embodiments, the first feed stream comprises H₂ and CO₂, as these can be reacted in contact with a hydrogenation catalyst to produce methanol. Accordingly, in various embodiments as otherwise described herein, the first feed stream includes at least 10 mol% H₂. For example, in various embodiments, the first feed stream includes at least 20 mol% H₂, e.g., at least 30 mol% H₂. In various embodiments as otherwise described herein, the first feed stream includes at least 5 mol% CO₂. For example, in various embodiments, the first feed stream includes at least 10 mol% CO₂, e.g., at least 15 mol% CO₂.

In general, CO₂ hydrogenation to produce methanol proceeds according to the reaction: CO₂ + 3H₂ → CH₃OH + H₂O. Accordingly, in various embodiments as otherwise described herein, the ratio of H₂:CO₂ of the first feed stream is at least 1:1, e.g., at least 1.5:1, on a molar basis. For example, in various embodiments as otherwise described herein, the ratio of H₂:CO₂ of the first feed stream is at least 2:1, e.g., at least 2.5:1, or at least 3:1, or at least 4:1, or at least 5:1, or at least 6:1, or at least 8:1. In particular embodiments, the ratio of H₂:CO₂ of the first feed stream is no more than 25:1, e.g., no more than 20:1, or no more than 15:1, or no more than 12:1.

In some embodiments, the first feed stream comprises other gases in addition to CO₂ and H₂. For example, in various embodiments, the first feed stream further comprises one or more of CO, CH₄, and N₂. For example, in particular embodiments, the first feed stream comprises other gases in addition to CO₂ and H₂ (e.g., one or more of CO, CH₄, and N₂) in an amount up to 70 mol%. In various embodiments, the first feed stream comprises no more than 1% O₂, for example, no more than 0.1% O₂, or no more than 0.01 % O₂, or substantially no O₂.

As otherwise described herein, the first feed stream is contacted with a hydrogenation catalyst. The hydrogenation catalyst may be selected by the person of ordinary skill in the art. Examples of suitable catalysts include Cu/ZnO catalysts, for example supported on aluminum oxide or zirconium oxide.

Advantageously, the CO₂ hydrogenation reaction may be performed at a relatively low temperature, leading to increased energy efficiency and integration of the overall process. For example, in various embodiments as otherwise described herein, the contacting of the first feed stream with the hydrogenation catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C. In various embodiments, the contacting of the first feed stream with the hydrogenation catalyst is performed at a pressure of no more than 100 bar, for example, no more than 80 bar, or no more than 60 bar.

The CO₂ hydrogenation caused by contacting the first feed stream with the hydrogenation catalyst produces a first product stream. As described herein, the formation of the first product stream advantageously produces methanol with high selectivity. Accordingly, in various embodiments as otherwise described herein, the formation of the first product stream is performed with a selectivity of at least 50% for methanol, e.g., at least 55%, or at least 60%. For example, in particular embodiments, the formation of the first product stream is performed with a selectivity of at least 65% for methanol (e.g., at least 70%, or at least 75%, or at least 80% for methanol). Advantageously, the formation of the first product stream can be performed with a low selectivity for methane. In various embodiments as otherwise described herein, the formation of the first product stream is performed with a selectivity of no more than 20% for methane, e.g., no more than 10%, or no more than 5% for methane. As the person of ordinary skill in the art will understand, "selectivity" for a given species is the fraction of the carbon-containing reactant that reacts that is converted to that species, i.e., not counting unreacted material.

Importantly, the CO₂ hydrogenation reaction used to form the first product stream as described herein is not a reverse water-gas shift reaction. As known in the art, the reverse water-gas shift reaction transforms CO₂ and H₂ into CO and H₂O. Accordingly, in various embodiments as otherwise described herein, the formation of the first product stream is performed with a selectivity of no more than 20% for CO, e.g., no more than 10%, or no more than 5% for CO. In particular embodiments, the formation of the first product stream is performed with a selectivity of no more than 2% for CO, or 1% for CO.

During the CO₂ hydrogenation reaction used to form the first product stream, at least a portion of the CO₂ is hydrogenated into MeOH and H₂O. Advantageously, this process may be performed with a relatively high conversion of CO₂. Accordingly, in various embodiments as otherwise described herein, the formation of the first product stream is performed with a conversion of CO₂ of at least 25%, e.g. at least 35%, e.g., at least 45% or at least 50%.

The person of ordinary skill in the art will be familiar with catalytic methods for the hydrogenation of carbon dioxide to methanol. Various examples of catalysts and catalytic processes are described in R.Guil-López et al., Materials, 12, 3902 (2019); Xiao et al., In: Aresta et al. (eds) An Economy Based on Carbon Dioxide and Water (2019); Marlin et al., Front. Chem. (2018); Rodriguez et al., ACS Cat. 5(11), 6696 (2015); Choundhury, Chem. Cat. Chem. 4(5), 609 (2012), each of which is hereby incorporated herein by reference in its entirety.

In various embodiments as otherwise described herein, the first product stream includes at least 15 mol% methanol, e.g., at least 25 mol%, or at least 35 mol% methanol. As described herein, the CO₂ hydrogenation also has advantageously low selectivity for other products such as methane and/or carbon monoxide. Accordingly, in various embodiments as otherwise described herein, the first product stream includes no more than 10 mol% methane, e.g., no more than 5 mol% methane, or no more than 2 mol% methane. In various embodiments as otherwise described herein, the first product stream includes no more than 10 mol% CO, e.g., no more than 5 mol% CO, or no more than 2 mol% CO. Of course, in some embodiments, there may be more methane and/or CO present, for example, when either or both are provided as part of the first feed stream, e.g., as recycle from the Fischer-Tropsch output.

As described above, through CO₂ hydrogenation the contacting of the first feed stream with the hydrogenation catalyst produces both methanol and water. The formed water can in some cases be deleterious to subsequent processes. For example, excess water present in a subsequent methanol decomposition process step can undesirably cause CO to be converted back to CO₂ through the water-gas shift reaction. Accordingly, in various embodiments as otherwise described herein, the process further comprises separating at least a portion of water of the first product stream therefrom. For example, in particular embodiments, the process further comprises separating at least 50%, or at least 75%, or at least 90%, or at least 95%, or at least 99%, or at least 99.5% of the water of the first product stream therefrom. In various embodiments as otherwise described herein, the portion of the first product stream that is included in the second feed stream has a water content of no more than 10 mol%, e.g., no more than 2 mol%, or no more than 1 mol%, or no more than 0.5 mol%.

In embodiments in which water is separated from the first product stream, the water may be disposed of as waste, or recycled into other processes. For example, in various embodiments, the separated water is directed to an electrolysis reactor for the formation of H₂ gas, e.g., for use in this integrated process or in other processes. In various embodiments, the separated water is subjected to a purification step before introduction into the electrolysis reactor.

In various embodiments as otherwise described herein, not all H₂ gas input to the CO₂ hydrogenation is reacted. Accordingly, in such embodiments, the process may further comprise separating at least a portion of H₂ from the first product stream. For example, in particular embodiments as otherwise described herein, the process further comprises separating at least 50%, or at least 60%, or at least 75%, or at least 90%, or at least 95% of the H₂ in the first product stream. The separated H₂ may be optionally purified, and then optionally recycled. In various embodiments, the at least a portion of the separated H₂ may be recycled to the first feed stream, and/or may be directed to another reactor. For example, in various embodiments, the at least a portion of the separated H₂ is directed to a Fischer-Tropsch reactor feed stream (e.g., at least a portion of the H₂ separated from the first product stream is provided to the third feed stream). In particular embodiments, the process further comprises activating the Fischer Tropsch catalyst using at least a portion of the H₂ separated from the first product stream. Additionally or alternatively, at least a portion of the H₂ separated from the first product stream may be used to activate a methanol decomposition catalyst.

Compared to gaseous compositions, compositions that are liquid at ambient temperature and pressure generally possess lower capital expenditure for handling due to their increased density and decreased need for high pressures and/or low temperatures. The present inventors have noted that the methanol product of the CO₂ hydrogenation can be simply stored and/or transported. Accordingly, in various embodiments as otherwise described herein, the contacting of the second feed stream and/or third feed stream with the methanol decomposition catalyst and the contacting of the first feed stream with the hydrogenation catalyst may be performed in different plants. For example, in some embodiments, the contacting of the second feed stream and/or third feed stream with the methanol decomposition catalyst and the contacting of the first feed stream with the hydrogenation catalyst are performed in different plants. As used herein, a different "plant" is not merely a different catalyst bed or different vessel, but rather a different facility than the facility in which the carbon dioxide hydrogenation is performed. In such embodiments, the plant in which the second feed stream and/or third feed stream is contacted with the methanol decomposition catalyst can be, e.g., at least 1 km away from the plant in which the first feed stream is contacted with the hydrogenation catalyst.

In various embodiments as otherwise described herein, a synthesized methanol fraction of the second feed stream is substantially greater than a synthesized methanol fraction of the third feed stream. As used herein, the term "synthesized methanol" signifies methanol that is provided substantially directly to methanol decomposition, without storage to significantly delay its use. "Synthesized methanol" is methanol that is contacted with a methanol decomposition catalyst under methanol decomposition conditions within a first characteristic process time of its synthesis from hydrogenation of CO₂ as described herein. The synthesized methanol may be transported from a CO₂ hydrogenation reactor to a methanol decomposition reactor, such as via pipeline, railcar, or truck. The "synthesized methanol fraction" of a feed stream is the fraction of methanol of the feed stream that is "synthesized methanol" as described above. As used herein, "stored methanol" is methanol that is made by hydrogenation of CO₂ as described herein, and is contacted with a methanol decomposition catalyst under methanol decomposition conditions only after the lapse of a first characteristic process time from its synthesis from hydrogenation of CO₂. The "stored methanol fraction" of a feed stream is the fraction of methanol of the feed stream that is "stored methanol" as described above.

The first characteristic process time is a value that distinguishes between substantially immediate use of methanol from a CO₂ hydrogenation on one hand, and the longer-term storage of such methanol on the other hand. This value can be selected by the person of ordinary skill in the art based on the present description, taking into account a variety of factors, for example, the size of the plant, the distance between a CO₂ hydrogenation reactor and a methanol decomposition reactor, and the potential for intermittency of provision of hydrogen for the synthesis of methanol. In various embodiments as otherwise described herein, the first characteristic process time is in the range of 30 minutes to 4 hours. For example, in various embodiments the first characteristic process time is in the range of 30 minutes to 3.5 hours, e.g., 30 minutes to 3 hours, or 30 minutes to 2.5 hours, or 30 minutes to 2 hours, or 30 minutes to 1.5 hours, or 30 minutes to 1 hour. In various embodiments as otherwise described herein, the first characteristic process time is in the range of 1-4 hours, e.g., 1-3.5 hours, or 1-3 hours, or 1-2.5 hours, or 1-2 hours, or 1-1.5 hours. In various embodiments as otherwise described herein, the first characteristic process time is in the range of 1.5-4 hours, e.g., 1.5-3.5 hours, or 1.5-3 hours, or 1.5-2.5 hours, or 1.5-2 hours. In various embodiments as otherwise described herein, the first characteristic process time is in the range of 2-4 hours, e.g., 2-3.5 hours, or 2-3 hours, or 2-2.5 hours. In various embodiments as otherwise described herein, the first characteristic process time is in the range of 2.5-4 hours, e.g., 2.5-3.5 hours, or 2.5-3 hours. In various embodiments as otherwise described herein, the first characteristic process time is in the range of 3-4 hours, e.g., 3-3.5 hours, or 3.5-4 hours. In various embodiments as otherwise described herein, the first characteristic process time is 30 minutes. In various embodiments as otherwise described herein, the first characteristic process time is 1 hour. In various embodiments as otherwise described herein, the first characteristic process time is 1.5 hours. In various embodiments as otherwise described herein, the first characteristic process time is 2 hours. In various embodiments as otherwise described herein, the first characteristic process time is 2.5 hours. In various embodiments as otherwise described herein, the first characteristic process time is 3 hours. In various embodiments as otherwise described herein, the first characteristic process time is 3.5 hours. In various embodiments as otherwise described herein, the first characteristic process time is 4 hours.

The synthesized methanol fraction of a feed stream is determined at time of contact with the methanol decomposition catalyst, as the fraction of total methanol that is synthesized methanol. Thus, a feed stream having 10 kg of methanol provided directly from CO₂ hydrogenation (i.e., within a first characteristic process time such as 2 hours) and 40 kg of methanol stored from much earlier CO₂ hydrogenation would have a synthesized methanol fraction of 0.20.

As described above, the present inventors have recognized that methanol can serve as a storage vehicle for CO and H₂, as it can be easily transported and stored as a liquid. Thus, stored methanol can serve as a buffer against intermittent syngas production. Thus, methanol can be synthesized (e.g., by CO₂ hydrogenation), or otherwise provided, and stored for later use, acting essentially as a stockpile of stored CO and H₂. During times when hydrogen production is high, such as in some embodiments during the first period of time, a portion of the methanol made by CO₂ hydrogenation may be stored for later use. Accordingly, in various embodiments as otherwise described herein, the method further comprises, during the first period of time, storing at least a portion of the methanol of the first product stream. In various embodiments as otherwise described herein, at least a portion of the methanol stored during the first period of time is provided to the third feed stream during the second period of time.

For example, in various embodiments as otherwise described herein, the process further comprises storing at least a portion of the methanol of the first product stream to provide stored methanol. Methanol can be stored, e.g.,, in various embodiments as otherwise described herein, in a storage tank, railcar, pipeline under stopped flow conditions, or other suitable holding vessel.

In various embodiments as otherwise described herein, the first period of time can be characterized by abundant hydrogen production such that methanol can be directly synthesized and then subjected to decomposition to produce an H₂/CO stream, and/or diverted to storage for later use. In various embodiments, H₂ production through electrolysis, and therefore methanol production, may vary in proportion to the availability of renewable sources of energy, such a wind or solar. For example, in various embodiments, synthesized methanol of the first product stream may be divided between the second feed stream and a storage stream, wherein the storage stream transmits the synthesized methanol to storage. In time periods when methanol production is high, synthesized methanol may be provided to both the second feed stream and to the storage stream. In embodiments where methanol production is moderate, for example commensurate with the demands of a downstream process, such as Fischer-Tropsch hydrocarbon production, synthesized methanol may be provided to the second feed stream, and essentially no methanol provided to the storage stream. In embodiments where methanol production is low, for example below the demands of a downstream process, such as Fischer-Tropsch hydrocarbon production, synthesized methanol may be provided to the second feed stream, for example, all synthesized methanol may be provided to the second feed stream. In such embodiments, stored methanol may be also provided to the second feed stream, for example sufficient stored methanol to meet the needs of a downstream process when provided in combination with the synthesized methanol. In embodiment where methanol production is essentially zero, no synthesized methanol may be provided to the second feed stream, and stored methanol may be supplied to the second feed stream.

Accordingly, in various embodiments as otherwise described herein, substantially all of the methanol of the second feed stream is synthesized methanol. For example, in various embodiments, the second feed stream has a synthesized methanol fraction of at least 95%, e.g., at least 98%, or at least 99%. However, substantial amounts of methanol other than synthesized methanol may be present in the second feed stream in some embodiments. For example, in various embodiments, the second feed stream has a synthesized methanol fraction of at least 50%, e.g., at least 65%, or at least 75%. In various embodiments, the second feed stream has a synthesized methanol fraction of at least 80%, e.g., at least 85%, or at least 90%.

In various embodiments as otherwise described herein, during a second period of time a third feed stream comprising stored methanol is provided to the methanol decomposition catalyst. Advantageously, stored methanol can be used during times when the supply of hydrogen is relatively lower than in the first period of time, such as times when energy production from renewable sources is low. In such times, the output of synthesized methanol may be decreased and stored methanol used to continue a relatively constant methanol supply to the methanol decomposition catalyst, thereby enabling the maintenance of a desirable production of an H₂/CO stream. Accordingly, in various embodiments as otherwise described herein, the third feed stream has a synthesized methanol fraction of no more than 90%, e.g., no more than 85%, or no more than 80%. In various embodiments, the third feed stream has a synthesized methanol fraction of no more than 75%, e.g., no more than 70%, or no more than 65 wt%. In various embodiments, the third feed stream has a synthesized methanol fraction of no more than 60 wt%, e.g., no more than 55 wt%, or no more than 50 wt%. In various embodiments, the third feed stream has a synthesized methanol fraction of no more than 45%, e.g., no more than 40%, or no more than 35%. In various embodiments, the third feed stream has a synthesized methanol fraction of no more than 30%, e.g., no more than 25%, or no more than 20%. In various embodiments, the third feed stream has a synthesized methanol fraction of no more than 15%, e.g., no more than 10%, or no more than 5%.

Substantial amounts of methanol of the third feed stream can be provided by stored methanol. For example, in various embodiments, substantially all of the methanol of the third feed stream is stored methanol. But amounts can vary. In various embodiments, the third feed stream has a stored methanol fraction of at least 5%, e.g., at least 10%, or at least 15%. In various embodiments, the third feed stream has a stored methanol fraction of at least 20%, e.g., at least 25%, or at least 30%. In various embodiments, the third feed stream has a stored methanol fraction of at least 35%, e.g., at least 40%, or at least 45%. In various embodiments, the third feed stream has a stored methanol fraction of at least 50%, e.g., at least 55%, or at least 60%. In various embodiments, the third feed stream has a stored methanol fraction of at least 65%, e.g., at least 70%, or at least 75%. In various embodiments, the third feed stream has a stored methanol fraction of at least 80%, e.g., at least 85%, or at least 90%. In various embodiments, the third feed stream has a stored methanol fraction of at least 95%, e.g., at least 98%, or at least 99%.

Stored methanol can conveniently be stored for substantial periods of time. The storage time of a body of methanol is the weight-average storage time over the body, e.g., a sample that includes 1 kg of methanol stored for 5 hours and 1 kg of methanol stored for 25 hours would have a storage time of 15 hours. A storage tank is assumed for this purpose to be homogenously mixed.

In various embodiments as otherwise described herein, stored methanol of the third feed stream has a storage time of at least three hours, e.g., at least 5 hours, or at least 8 hours. In various embodiments, stored methanol of the third feed stream has a storage time of at least 12 hours, e.g., at least 18 hours or at least 24 hours. In various embodiments, stored methanol has a storage time of at least 36 hours, e.g., at least three days or at least seven days.

Of course, while it can be desirable to store methanol for use when methanol production by hydrogenation of CO2 is reduced, it can also be desirable not to stockpile too much methanol. Accordingly, especially when the reduced methanol production is a cyclical phenomenon (e.g., as when depending on solar or tidal power), it can be desirable to use stored methanol reasonably regularly, so as to avoid stockpiling too much. Accordingly, in various embodiments, stored methanol of the third feed stream has a storage time of no more than 30 days, e.g., no more than 21 days, or no more than 14 days, or no more than seven days. In various embodiments, stored methanol of the third feed stream has a storage time of no more than three days, e.g., no more than two days, or no more than one day.

In some desirable embodiments the second feed stream and/or the third feed stream are substantially made up of synthesized methanol and stored methanol. For example, in various embodiments, the second feed stream and/or the third feed stream has a sum of a synthesized methanol fraction and a stored methanol fraction of at least 95%, e.g., at least 98%, or at least 99%. However, methanol of the second feed stream and/or the third feed stream can be provided by other sources, and so in some cases the sum of the synthesized methanol fraction of the second feed stream and/or the third feed stream is substantially less than 100%.

Stored methanol can be stored for significant amounts of time.

As described herein, in various embodiments, the synthesized methanol fraction of the second feed stream is substantially greater than the synthesized methanol fraction of the third feed stream. For example, in various embodiments, a difference between the synthesized methanol fraction of the second feed stream and the synthesized methanol fraction of the third feed stream is at least 5%, e.g., at least 10%, or at least 15%. In various embodiments, a difference between the synthesized methanol fraction of the second feed stream and the synthesized methanol fraction of the third feed stream is at least 20%, e.g., at least 25%, or at least 30%. In various embodiments, a difference between the synthesized methanol fraction of the second feed stream and the synthesized methanol fraction of the third feed stream is at least 35%, e.g., at least 40%, or at least 45%. In various embodiments, a difference between the synthesized methanol fraction of the second feed stream and the synthesized methanol fraction of the third feed stream is at least 50%, e.g., at least 55%, or at least 60%. In various embodiments, a difference between the synthesized methanol fraction of the second feed stream and the synthesized methanol fraction of the third feed stream is at least 65%, e.g., at least 70%, or at least 75%.

The processes described herein can be especially useful in addressing intermittent loss of hydrogen used to make methanol via CO₂ hydrogenation, for example, due to an intermittent decrease or loss of renewable sources of energy used to make the hydrogen. This can occur, e.g., due to a loss of solar power, for example, at night or during cloudy weather, or a loss of wind power, or a loss of tidal power. In such cases, stored methanol can be used to maintain the methanol decomposition process, e.g., during the second period of time. In various embodiments, the second period of time is in the range of 30 minutes to 24 hours in length (e.g., 30 minutes to 18 hours, or 30 minutes to 12 hours, or 1-24 hours, or 1-18 hours, or 1-12 hours, or 3-24 hours, or 3-18 hours, or 3-12 hours, or 6-24 hours, or 6-18 hours, or 6-12 hours), and occurs after the first period of time.

After the second period of time, when a renewable source of energy is restored, the use of stored methanol can be reduced or ceased. Accordingly, in various embodiments, the second period of time is followed by another first period of time (i.e., in which the synthesized methanol fraction of the second feed stream is substantially greater than the synthesized methanol fraction of the third feed stream as used during the second period of time). substantially greater than in the within 20% (e.g., within 10% of) of the synthesized/stored ratio of the first period of time.

In various embodiments, e.g., especially when the loss of renewable sources of energy is due to a regular phenomenon such as nightfall or tidal patterns, the process includes a plurality of alternating first and second periods of time.

In various embodiments as otherwise described herein, at least a portion of the second product stream may be recycled to the first feed stream. For example, in some embodiments, wherein the supply of methanol is low, it can be advantageous to maintain the operation of the methanol synthesis reactor.

As described herein, an advantage of the present disclosure is the ability to store and use intermittently-produced methanol. One advantage of methanol storage is that, as a liquid at atmospheric temperatures and pressures, it can be more easily and densely stored and transported, such as by pumping through a low-pressure pipeline. Accordingly, liquid methanol may often be more efficiently stored compared to many gaseous feed components, such as hydrogen (and/or carbon monoxide or carbon dioxide). Thus, storage of methanol, with the capability to product an H₂/CO stream from subsequent methanol decomposition, can obviate the need to store hydrogen gas (and/or carbon monoxide or carbon dioxide) for times of low production. Accordingly, in various embodiments as otherwise described herein, the hydrogen produced by electrolysis is not stored for a substantial amount of time. For example, in particular embodiments, the hydrogen produced by electrolysis is not stored for more than six hours, e.g., not stored for more than 3 hours, or more than 1 hours, or more than 30 minutes. For example, hydrogen is often stored in high pressure tanks and other vessels, which may be advantageously avoided.

During times when hydrogen production is high, such as times when any relevant renewable sources of energy production are abundant, it may be desirable to allow a portion of the hydrogen to bypass the hydrogenation reaction and be provided to the H₂/CO stream. Accordingly, in various embodiments as otherwise described herein, such as during the first period of time, the process further comprises providing at least a portion of the hydrogen to the H₂/CO stream. For example, in various embodiments, such as during the first period of time, the H₂/CO stream further comprises at least a portion of hydrogen not derived from methanol decomposition. In some embodiments, the portion of hydrogen not derived from methanol decomposition may include at least one of green hydrogen and/or blue hydrogen. In particular embodiments, the portion of hydrogen not derived from methanol decomposition may include grey hydrogen. For example, hydrogen from sources beyond methanol decomposition may be added to adjust the syngas composition in preparation for use by a further process (e.g., Fischer-Tropsch hydrocarbon synthesis).

As described herein, carbon monoxide may be utilized for methanol synthesis through hydrogenation. However, in various embodiments, the carbon monoxide of the first feed stream is limited, e.g., no more than 10 vol%. Accordingly, in various embodiments as otherwise described herein, carbon monoxide is provided directed to the H₂/CO stream. For example, in various embodiments, the H₂/CO stream further comprises at least a portion of carbon monoxide not derived from methanol decomposition. In some embodiments, carbon monoxide from sources beyond methanol decomposition may be added to adjust the syngas composition in preparation for use by a further process (e.g., Fischer-Tropsch hydrocarbon synthesis).

In various embodiments as otherwise described herein, the second feed stream and/or third feed stream introduces methanol to a methanol decomposition catalyst for decomposition into CO and H₂. Methanol decomposition is a distinct process from methanol reforming. In methanol reforming, methanol and water are reacted to produce CO₂ and H₂. Accordingly, in various embodiments as otherwise described herein, methanol reforming is not utilized. For example, in particular embodiments, no more than 10% of the H₂ is derived from methanol reforming (e.g., no more than 5%, or no more than 1%).

The water-gas shift reaction is another method to generate H₂ by reacting carbon monoxide with water. The reaction is reversible as well, where carbon dioxide may be reacted with hydrogen to form carbon monoxide and water. Advantageously, in various embodiments, the presently disclosed process can be used to avoid the use of both the water-gas shift reaction and reverse water-gas shift reaction. Accordingly, in various embodiments as otherwise described herein, no more than 10% (e.g., no more than 5%, or no more than 2%) of each of the H₂, CO, and CO₂ of the process are generated by the water-gas shift reaction or reverse water-gas shift reaction, as appropriate. In various processes as disclosed herein, there is no use of a dedicated water-gas shift reactor or reverse water-gas shift reactor (however, as would be appreciated by the person of ordinary skill in the art, the water-gas shift reaction or reverse water-gas shift reaction can operate to a minor degree as a side reaction during certain processes).

As described herein, methanol decomposition is utilized to form an H₂/CO stream that includes CO and H₂. Accordingly, in various embodiments as otherwise described herein, the second feed stream comprises at least 5 mol% methanol. For example, in particular embodiments, the second feed stream comprises at least 7.5% methanol, e.g., at least 10% methanol, or at least 15% methanol, or at least 20% methanol, or at least 25 mol% methanol. In times of intermittent availability of methanol, it may be desirable to maintain the process in operation. Accordingly, in various embodiments, the method further comprises contacting at least at least a portion of the H₂/CO stream with the hydrogenation catalyst (e.g., in the hydrogenation reactor).

Optionally, the second feed stream and/or third feed stream may also comprise one or more additional gases. In such embodiments, the second feed stream may further comprise one or more of H₂, CO, CH₄, CO₂, and N₂. In particular embodiments, the second feed stream comprises an inert carrier gas, wherein the inert carrier gas includes one or more of CH₄, CO₂ and N₂. Additionally or alternatively, H₂ and/or CO may be added to the second feed stream in order to tune the decomposition reaction and/or provide a desired second product stream for use in the Fischer-Tropsch process. In such embodiments, the second feed stream further comprises H₂ and/or CO. In various embodiments as otherwise described herein, one or more of H₂, CO, CH₄, CO₂, and N₂ are be present in second feed stream an amount in the range of up to 50 mol%, e.g., up to 40 mol%, or up to 30 mol%.

In various embodiments, the second feed stream and/or third feed stream has a low water content. Any water present can react with CO to be at least partially converted to CO₂ and H₂ through the water-gas shift reaction, and water can also react with methanol via reforming to provide H₂ and CO₂. The generated H₂, according to the reaction equilibrium of methanol decomposition, would then disfavor the decomposition of methanol to CO and H₂. Accordingly, in various embodiments as otherwise described herein, the second feed stream has a concentration of water of no more than 5 mol%, e.g., no more than 2 mol%, or no more than 1 mol %, or no more than 0.5 mol%, or no more than 0.3 mol%, or no more than 0.2 mol%, or no more than 0.1 mol%.

In certain embodiments, the second feed stream and/or third feed stream may be provided at elevated temperature. For example, in various embodiments as otherwise described herein, the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.

As described herein, the second feed stream and/or third feed stream is contacted with a methanol decomposition catalyst. Any suitable decomposition catalyst may be used; a variety are known in the art. For example, the decomposition catalyst may include a transition metal, for example, Ni, Co, Rh, Ir, Cu, Pt, Ru, or mixtures thereof. The decomposition catalyst may be a supported catalyst, wherein the support is a refractory oxide, such as oxidized diamond, silica, zirconia, ceria, titania, alumina, or magnesia. In some embodiments, the methanol decomposition catalyst is a copper/zinc oxide catalyst, e.g., on alumina.

The contacting of the second feed stream and/or third feed stream with the methanol decomposition catalyst occurs at a temperature suitable to effect efficient methanol decomposition. In various embodiments as otherwise described herein, the contacting of the second feed stream with the methanol decomposition catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C. The contacting may be performed at a variety of suitable pressures, for example, in the range of 0 to 50 barg, wherein barg is a unit of gauge pressure, being the pressure in bars above ambient or atmospheric pressure. In various embodiments as otherwise described herein, the contacting with the methanol decomposition catalyst may be integrated with a Fischer-Tropsch reactor. Accordingly, in such embodiments, the contacting with the methanol decomposition catalyst may be performed at relatively high pressures, for example, in the range of up to 50 barg, e.g., 20 to 40 barg.

As described herein, the contacting of the second feed stream and/or third feed stream with the methanol decomposition catalyst may be performed in the same plant as the contacting of the first feed stream with the hydrogenation catalyst. In such examples, it may be advantageous to coordinate the temperatures of the methanol decomposition reaction and the hydrogenation reaction in order to avoid excessive heating and cooling capital costs and energy costs. Accordingly, in particular embodiments as otherwise described herein, the contacting of the second feed stream with the methanol decomposition catalyst is performed at a temperature within 75 °C of a temperature of the contacting of the first feed stream with the hydrogenation catalyst, e.g., within 50 °C.

Methanol decomposition reaction generates CO and H₂, ideally with minimal other products. Accordingly, in various embodiments as otherwise described herein, the decomposition of methanol is performed with a carbon product selectivity of at least 50% for CO, e.g., at least 60%, or at least 70%, or at least 80%, or at least 90% for CO. Advantageously, in various embodiments, the decomposition of methanol is performed with a selectivity of no more than 20% for CO₂, e.g., no more than 15%, or no more than 10%, or no more than 5%. In various embodiments as otherwise described herein, the decomposition of methanol is performed with a conversion of methanol of at least 30%, e.g., at least 40%, or at least 50%, or at least 60%, or at least 65%, or at least 70%, or at least 75%.

As described herein, the decomposition of methanol, such as through the contacting of the second feed stream and/or third feed stream with a methanol decomposition catalyst, generates CO and H₂. Accordingly, in various embodiments as otherwise described herein, the second product stream comprises at least 20 mol% total of CO and H₂, e.g., at least 35 mol%, or at least 50 mol%, or at least 65 mol%. Without wishing to be bound by theory, methanol decomposition under these conditions is expected to generated two moles of H₂ per mole of CO. Accordingly, in various embodiments as otherwise described herein, the second product stream has a molar ratio of hydrogen to carbon monoxide in the range of 0.5:1 to 5:1, e.g., 1:1 to 3:1, or 1.5:1 to 2.5:1, or 1.5:1 to 3.5:1. Of course, in other embodiments, this molar ratio may be different, e.g., as a consequence of inclusion of H₂ and/or CO in the second feed stream and/or third feed stream.

Advantageously, a proportion of methanol is consumed during the methanol decomposition reaction. Accordingly, in various embodiments as otherwise described herein, the second product stream includes no more than 75 mol% methanol, e.g., no more than 60 mol% methanol, no more than 50 mol% methanol, or no more than 25 mol% methanol.

In some embodiments, further removal of methanol from the second product stream is desired. For example, in various embodiments as otherwise described herein, the process further comprises separating at least a portion of methanol from the second product stream, e.g., separating at least 50%, or at least 75%, or at least 90%, or at least 95% of the methanol from the second product stream. Advantageously, methanol of the second product stream, e.g., at least a portion of the methanol separated from the second product stream, may be transferred or recycled to other processes. For example, in various embodiments as otherwise described herein, the process further comprises recycling to the second feed stream at least a portion of the methanol separated from the second product stream. In other embodiments, the process further comprises storing at least a portion of the methanol from the second product stream to form recycled and stored methanol, and then providing at least a portion of the recycled and stored methanol to the third feed stream.

The person of ordinary skill in the art will be familiar with catalytic methods for the decomposition of methanol to CO and H₂. Various examples of catalysts and catalytic processes are described in U.S. Patent no. 6,541,142, U.S. Patent no, 9,883,773, and U.S. Patent no. 4,716,859 each of which is hereby incorporated herein by reference in its entirety.

Methanol is a valuable feedstock for a variety of chemical processes. And when made using carbon dioxide from renewable sources using the processes described herein (e.g., capture from atmosphere or other chemical processes, or biomass conversion), it can be advantageously be considered as a carrier for renewable carbon. Similarly, when made using green hydrogen and/or blue hydrogen as described herein (e.g., hydrogen formed from water electrolysis or steam reforming), the methanol can be considered as a carrier of hydrogen from such sources.

The methanol decomposition reaction produces CO and H₂ in a theoretical 2:1 molar ratio, although in various embodiments the actual reaction output may vary. This can result in excess hydrogen useful for subsequent processes, depending on the desired ratio. And, notably, hydrogen may be present in the second feed stream, further increasing the amount of hydrogen in the second product stream. Accordingly, in various embodiments as otherwise described herein, the process further comprises separating at least a portion of H₂ from the second product stream. The separated H₂ may be suitable for a variety of processes. For example, in particular embodiments, at least a portion of the separated H₂ from the second product stream is provided to the first feed stream. Additionally or alternatively, in various embodiments as otherwise described herein, the process further comprises activating the methanol decomposition catalyst or the Fischer-Tropsch catalyst using at least a portion of the H₂ separated from the second product stream.

As described herein, in various embodiments the methanol decomposition results in products containing 2:1 molar ratios of hydrogen gas and carbon monoxide. However, this ratio may be not be ideal for downstream processes. For example, Fischer-Tropsch hydrocarbon synthesis (e.g., for the formation of C₅₊ hydrocarbons) typically requires a molar ratio of hydrogen to carbon monoxide in the range of 1.8:1 to 4:1. Accordingly, in various embodiments as otherwise described herein, the process further comprises adjusting the syngas to provide a H₂/CO stream with a H₂:CO ratio in the range of 1.5:1 to 3:1.

The adjusting may be accomplished through a variety of means, including combinations thereof. For example, in various embodiments, the adjusting comprises subjecting at least a portion of the H₂/CO stream to the water-gas shift reaction (e.g., in a water-gas shift reactor). In various embodiments, the adjusting comprises adding hydrogen gas to the H₂/CO stream. For example, wherein the process comprises electrolyzing water to produce a hydrogen gas containing stream (e.g., as otherwise described herein), and using at least a portion of the hydrogen gas containing stream in the adjusting step. In various embodiments, the adjusting comprises removing carbon monoxide from the H₂/CO stream.

As described herein, as aspect of the present disclosure is the production of syngas. In various embodiments, the production of syngas proceeds through the production of methanol from carbon dioxide, followed by methanol decomposition to produce carbon monoxide and hydrogen. Accordingly, the syngas may further include gases beyond carbon monoxide and hydrogen. For example, in various embodiments as otherwise described herein, the syngas comprises methane in an amount in the range of 0.1 wt% to 50 wt% (e.g., 1 wt% to 50 wt%, 5 wt% to 50 wt%, 10 wt% to 50 wt%, 15 wt% to 50 wt%, 20 wt% to 50 wt%, 25 wt% to 50 wt%, 0.1 wt% to 40 wt%, 0.1 wt% to 30 wt%, 0.1 wt% to 25 wt%, 0.1 wt% to 20 wt%, 1 wt% to 30 wt%, 2 wt% to 30 wt%, 5 wt% to 30 wt%). For example, in various embodiments as otherwise described herein, the syngas comprises carbon dioxide in an amount in the range of 0.1 wt% to 50 wt% (e.g., 1 wt% to 50 wt%, 5 wt% to 50 wt%, 10 wt% to 50 wt%, 15 wt% to 50 wt%, 20 wt% to 50 wt%, 25 wt% to 50 wt%, 0.1 wt% to 40 wt%, 0.1 wt% to 30 wt%, 0.1 wt% to 25 wt%, 0.1 wt% to 20 wt%, 1 wt% to 30 wt%, 2 wt% to 30 wt%, 5 wt% to 30 wt%).

Certain processes, such as a methanol synthesis process such as CO₂ hydrogenation, may suffer from reduced efficiency if given variable input quantities or periodically idled. Accordingly, in various embodiments, the H₂/CO stream may be recycled to the hydrogenation reactor in order to continue methanol synthesis. For example, the process as otherwise described herein may further include contacting at least a portion of the H₂/CO stream with the hydrogenation catalyst (e.g., in the hydrogenation reactor).

The H₂/CO stream of the present disclosure may be utilized by a variety of downstream processes. Accordingly, in another aspect, the present disclosure provides for processes for the production of hydrocarbons, the process comprising performing a Fischer-Tropsch reaction by contacting at least a portion of the H₂/CO stream as otherwise described herein with a Fischer-Tropsch catalyst (e.g., in a Fischer-Tropsch reactor) to provide a Fischer-Tropsch product stream comprising hydrocarbons, water, and optionally oxygenated hydrocarbons.

In embodiments utilizing a Fischer-Tropsch reaction, the operation of the process as otherwise described herein may be modulated in relation to the requirements of the Fischer-Tropsch reaction. For example, in various embodiments as otherwise described herein, wherein the Fischer-Tropsch reaction has a desired throughput, and wherein during the first period of time, a supply of electricity from a renewable source is sufficient to provide the desired throughput, and wherein during the second period of time, the supply of electricity from a renewable source is insufficient to provide the desired throughput.

The Fischer-Tropsch synthesis catalyst may be any suitable known in the art. For example, the Fischer-Tropsch synthesis catalyst may be an iron-based catalyst or a cobalt-based catalyst. Other examples of suitable catalysts include those based on nickel or platinum. A person of ordinary skill in the art may select a suitable catalyst in light of the present disclosure.

The Fischer-Tropsch synthesis catalyst (e.g., supported Fischer-Tropsch synthesis catalyst) used in various embodiments of the processes of the disclosure comprises in the range of 1 wt% to 35 wt% iron or cobalt on an elemental basis. The person of ordinary skill in the art will, based on the disclosure herein, select a suitable amount of iron or cobalt. For example, in various embodiments, the Fischer-Tropsch synthesis catalyst comprises cobalt in an amount in the range of 1-30 wt%, or 1-25 wt%, or 1-20 wt%, or 2-35 wt%, or 2-30 wt%, or 2-25 wt%, or 2-20 wt%, or 5-35 wt%, or 5-30 wt%, or 5-25 wt%, on an elemental basis. In certain particular embodiments, the Fischer-Tropsch synthesis catalyst comprises cobalt in an amount in the range of 2-20 wt%, e.g., 2-15 wt%, or 2-10 wt%, or 5-20 wt%, or 5-15 wt%, or 5-10 wt%, or 7-20 wt%, or 7-15 wt%, on an elemental basis.

In certain embodiments, the suitable Fischer-Tropsch synthesis catalysts may also possess a wide variety of other transition metals. For example, a variety of promoters, such as one or more of manganese, ruthenium, palladium, platinum, rhodium, rhenium, chromium, nickel, iron, molybdenum, tungsten, zirconium, gallium, thorium, lanthanum, cerium, copper and mixtures thereof may be included. A promoter is typically used in a metal to promoter atomic ratio of up to 250:1, e.g., up to 125:1, or up to 25:1, or up to 10:1, wherein the metal is a catalytically active Fischer-Tropsch metal, such as iron or cobalt. In certain such embodiments, the one or more promoters are present in the Fischer-Tropsch synthesis catalyst obtained in an amount from 0.1 wt% to 3 wt%, on an elemental basis, based on the total weight of the supported synthesis catalyst. In other embodiments, the Fischer-Tropsch synthesis catalyst does not contain any such promoters.

The Fischer-Tropsch synthesis catalyst may further comprise a support material (e.g., the Fischer-Tropsch synthesis catalyst may be a supported Fischer-Tropsch synthesis catalyst). The support material serves to bind and structurally support the catalyst particles and may also influence the catalytic activity and product selectivity. In various embodiments as otherwise described herein, the support material includes at least one of alumina, zirconia, titania, silica, zinc oxide, ceria, or combinations thereof. In particular embodiments, the support material comprises one of alumina, zirconia, zinc oxide, ceria, silica and titania, for example the support material is one of alumina, zirconia, zinc oxide, ceria, silica and titania. In yet other particular embodiments, the support material comprises titania (e.g., at least 90 wt% titania, or at least 95 wt% titania, or at least 99 wt% titania), for example the support material is titania.

In various embodiments as otherwise described herein, the Fischer-Tropsch synthesis catalyst used in accordance with the present disclosure may be prepared by any suitable method. For example, the Fischer-Tropsch synthesis catalyst may be prepared by a process in which the cobalt is impregnated on to the support material.

A suitable impregnation method, for example, comprises impregnating a support material with metal-containing compound, which is thermally decomposable to the oxide form. Impregnation of the support material with the metal-containing compound may be achieved by any suitable method of which the skilled person is aware, for instance by vacuum impregnation, incipient wetness or immersion in excess liquid.

The incipient wetness technique is so-called because it requires that the volume of impregnating solution be predetermined so as to provide the minimum volume of solution necessary to just wet the entire surface of the support, with no excess liquid. The excess solution technique as the name implies, requires an excess of the impregnating solution, the solvent being thereafter removed, usually by evaporation.

In various embodiments, the support material, if present, may be in the form of a powder, granulate, shaped particle, such as a preformed sphere or microsphere, or extrudate. Reference herein to a powder or granulate of a support material is understood to refer to free flowing particles of a support material or particles of support material that have undergone granulation and/or sieving to be a particular shape (e.g. spherical) and size range. Reference herein to an "extrudate" is intended to mean a support material that has undergone an extrusion step and therefore may be shaped.

It will be understood that the support material may be in any form provided it is suitable for use as a support for a Fischer-Tropsch synthesis catalyst. Preferred support materials are substantially free of extraneous components which might adversely affect the catalytic activity of the system. Thus, in certain embodiments, support materials are at least 95 % w/w pure, more preferably at least 98 % w/w pure and most preferably at least 99 % w/w pure. Impurities preferably amount to less than 1% w/w, more preferably less than 0.50 % w/w and most preferably less than 0.25 % w/w. The pore volume of the support is preferably more than 0.150 ml/g and preferably more than 0.30 ml/g. The average pore radius (prior to impregnation) of the support material is 10 to 500 Å, preferably 15 to 100 Angstroms, more preferably 20 to 80 Å and most preferably 25 to 60 Å. The BET surface area is suitably from 2 to 1000 m²g, preferably from 10 to 600 m²/g, more preferably from 15 to 300 m²/g, and most preferably 30 to 150 m²/g.

The solvent of the impregnating solution(s) may be either an aqueous solvent or a non-aqueous, organic solvent. Suitable non-aqueous organic solvents include, for example, alcohols (e.g. methanol, ethanol and/or propanol), ketones (e.g. acetone), liquid paraffinic hydrocarbons and ethers. Alternatively, aqueous organic solvents, for example an aqueous alcoholic solvent, may be employed. Preferably, the solvent of the impregnating solution(s) is an aqueous solvent.

It will be appreciated that where the support material is in powder or granulate form, once impregnated with suitable metal compounds, such as a cobalt containing compound, iron-containing compound, or the like, the impregnated support material may be extruded or formed into shaped particles at any suitable stage before or after drying and calcining.

Impregnation of the support material is usually followed by drying of the impregnating solution in order to effect precipitation of the metal-containing compound on to the support material and preferably also to remove bound solvent of the impregnating solution (e.g. water). As will be appreciated, in embodiments where an extrusion is performed, complete drying and removal of solvent (e.g. bound solvent) of the impregnating solution may occur after forming of a shaped particle, for example by extrusion. Drying is suitably conducted at temperatures from 50 °C to 150 °C, preferably 75 °C to 125 °C. Suitable drying times are, for example, from 5 minutes to 72 hours. Drying may suitably be conducted in a drying oven or in a box furnace, for example, under the flow of an inert gas at elevated temperature.

Preparation of the Fischer-Tropsch synthesis catalyst may involve a calcination step. As will be understood, in certain embodiments as otherwise described herein, calcination is required for converting any metal-containing compound which has been impregnated on the support material into an oxide of the metal. For example, wherein the metal is cobalt, calcination leads to thermal decomposition of the cobalt-containing compound, and not merely removal of bound solvent of an impregnating solution, as for instance in the case of drying.

Calcination may be performed by any method known to those of skill in the art, for instance in a fluidized bed or rotary kiln at a temperature of at least 250 °C, preferably from 275 °C to 500 °C.

The Fischer-Tropsch synthesis catalyst may conveniently be subjected to reductive activation by any known means of which the skilled person is aware. For example, in embodiments wherein the catalyst comprises cobalt, activation conditions are such that are capable of converting cobalt oxide to the active cobalt metal (e.g., to form a reduced Fischer-Tropsch synthesis catalyst), such as through the use of a reducing gas. In particular embodiments, the reducing gas comprises hydrogen gas. The step of forming a reduced synthesis catalyst may be carried out batch wise or continuously in a fixed bed, fluidised bed or slurry phase reactor, or in-situ in the same reactor as will be subsequently used for the Fischer-Tropsch synthesis reaction. Reduction is suitably performed at a temperature of from 150 °C to 350 °C, e.g., from 150 °C to 325 °C, or from 200 °C to 325 °C, or from 200 °C to 300 °C, or from 200 °C to 250 °C.

In various embodiments as otherwise described herein, the process comprises electrolyzing water to produce hydrogen gas. In certain such embodiments, the process may further comprise activating the Fischer-Tropsch catalyst by contacting it with at least a portion of the hydrogen gas produced by the electrolysis of water, for example in the electrolyzing step as otherwise described herein.

As will be appreciated, in some embodiments the H₂/CO stream supplied to the Fischer-Tropsch synthesis catalyst may be used to form a reduced Fischer-Tropsch synthesis catalyst in situ, without requiring any preceding or distinct reductive activation step.

The person of ordinary skill in the art will perform the processes described herein using any desirable reaction systems. For example, a wide variety of reactors can be used, e.g., a fixed bed reactor, a slurry bed reactor, or a fluid bed reactor.

As used herein, "selectivity" for a given component is measured as the molar fraction of a particular reactant that is reacted in the process (i.e., not including any unreacted portion of that particular reactant) and is converted to that product. For example, in the reaction of carbon dioxide and hydrogen to provide product components including methane, "selectivity" for a given component is defined as the molar fraction of carbon dioxide that is reacted in the process and is converted to the product of interest, not including any unreacted carbon dioxide.

A method of utilizing carbon dioxide is the reverse water gas shift reaction, where carbon dioxide and hydrogen are converted to carbon monoxide and water:

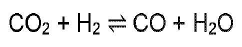

However, the reverse water gas shift reaction it typically performed at in extreme conditions, with reaction temperatures in excess of 900 °C. Thus, the reaction is costly from an energy standpoint, and specialized equipment must be used. Further, the process consumes hydrogen which is often expensive in process economics. An advantage of the processes of the present disclosure is the ability, in various embodiments, to convert carbon dioxide to syngas through a methanol intermediate without using the reverse water gas shift reaction. Accordingly, in various embodiments as otherwise described herein, the process does not include a reverse water gas shift reaction. It is possible that a small proportion of carbon dioxide are converted to carbon monoxide by the reverse water-gas shift reaction as a reaction side product during normal operation of the processes described herein. Accordingly, the absence of a reverse water-gas shift reaction is understood to mean that there is not a distinct reaction zone dedicated to the reverse water-gas shift reaction.

In other embodiments as otherwise described herein, the process includes a water gas shift reaction, however, in particular embodiments, the water gas shift reaction is operated so as to consume carbon monoxide and water and generate carbon dioxide and hydrogen.

As such, in various embodiments, the Fischer-Tropsch product stream may further comprise carbon dioxide. In such embodiments, the process may further comprise recycling at least a portion of the carbon dioxide to the first feed stream.

And as noted herein, mixtures of carbon monoxide and hydrogen are useful in the synthesis of higher hydrocarbons via the Fischer-Tropsch process. Accordingly, another aspect of the disclosure is a Fischer-Tropsch process comprising contacting a hydrocarbon synthesis mixture comprising carbon monoxide and hydrogen with a Fischer-Tropsch hydrocarbon synthesis catalyst to produce a hydrocarbon composition comprising C₅₊ hydrocarbons and/or oxygenates with a selectivity for C₅₊ hydrocarbons of at least 50% and/or a selectivity for oxygenates of at least 20%. Suitable techniques for hydrocarbon synthesis, especially with regard to the synthesis of C₅₊ hydrocarbons and/or oxygenates, are described in International Patent Application Publication No. 2019/154885, hereby incorporated by reference herein in its entirety

The Fischer-Tropsch process as otherwise described herein may also have an advantageously high C₅₊ selectivity. For example, in various embodiments, the process has a C₅₊ selectivity of at least 75%, for example, at least 80%, or at least 85%, or at least 90%. The Fischer-Tropsch process as otherwise described herein may also have an advantageously low methane selectivity. For example, in various embodiments, the process has a methane selectivity of no more than 6%, for example, no more than 5%, or 4%, or 3%.

CO conversion is defined as moles of CO used/moles of CO fed x 100. C₅₊ hydrocarbon selectivity is defined as moles of CO attributed to C₅₊ hydrocarbons/moles of CO converted x 100. Alcohol selectivity is defined as moles of CO attributed to alcohols/moles of CO converted x 100.

Conventional Fischer-Tropsch temperatures may be used in order to prepare liquid hydrocarbons in accordance with the present disclosure, using the catalysts described herein. For example, the temperature of the contacting of a mixture of hydrogen and the gaseous carbon oxide (e.g., in the form of a synthesis gas mixture) with a Fischer-Tropsch catalyst may suitably be in the range from 100 to 400 °C, such as from 100 to 350 °C, or 100 to 300 °C, or 100 to 250 °C, or 150 to 400 °C, or 150 to 350 °C, or 150 to 300 °C. In various embodiments, the contacting is conducted at a temperature of no more than 350 °C, e.g., no more than 325 °C, or no more than 300 °C, or no more than 280 °C. The pressure of the contacting (i.e., the temperature of the Fischer-Tropsch reaction) can in various embodiments suitably be in the range from 10 to 100 bara (from 1 to 10 MPa), such as from 15 to 75 bara (from 1.5 to 7.5 MPa), or from 20 to 50 bara (from 2.0 to 5.0 MPa). For example, in various embodiments the contacting is conducted at a pressure of no more than 7.5 MPa absolute.

In particular embodiments, the temperature of the Fischer-Tropsch reaction is in the range from 150 to 350 °C, more preferably from 180 to 300 °C. In preferred embodiments, the pressure of the Fischer-Tropsch reaction is in the range from 10 to 100 bar (from 1 to 10 MPa), more preferably from 10 to 60 bar (from 1 to 6 MPa) and most preferably from 20 to 45 bar (from 2 to 4.5 MPa).

In addition to hydrocarbons, the Fischer-Tropsch product stream may further include other components from the feed stream and/or formed *in situ,* such as light hydrocarbons (e.g., methane and/or C₂-C₄ hydrocarbons), nitrogen, water, and/or carbon dioxide. Advantageously, certain species may be separated and recycled to other processes. For example, in various embodiments as otherwise described herein, wherein the Fischer-Tropsch product stream comprises carbon dioxide, and wherein the process further comprises providing at least a portion of the carbon dioxide of the Fischer-Tropsch product stream to the first feed stream.

In various embodiments as otherwise described herein, the process comprises electrolyzing water to form a hydrogen gas containing stream. In such embodiments, and wherein the Fischer-Tropsch product stream comprises water, the process further comprises providing at least a portion of the hydrogen gas containing stream to the first feed stream, wherein at least a portion of the water of the Fischer-Tropsch product stream is provided to the electrolyzing step.

Unless otherwise stated, temperatures referred to in the Examples are applied temperatures and not catalyst/bed temperatures. Unless otherwise stated, pressures referred to in the Examples are absolute pressures.

The processes of the disclosure will now be further described by reference to the following Examples which are illustrative only.

An embodiment according to the present disclosure is shown in FIG. 1. Hydrolysis reactor 110 produces hydrogen stream 101, which is combined with carbon oxide (e.g., containing at least one of carbon dioxide and carbon monoxide) stream 102 to form first feed stream 103. First feed stream 103 is introduced into hydrogenation reactor 120 with hydrogenation catalyst 140 and the methanol-containing first product stream 104 is withdrawn. Depending on the time period as described herein, the synthesized methanol can be directly introduced into second feed stream 106 which enters methanol decomposition reactor 130 containing methanol decomposition catalyst 160, and second product stream 108 is withdrawn. Additionally or alternatively, methanol can be withdrawn from the first product stream 104 in stored methanol stream 105 to methanol storage tank 140. Subsequently, stored methanol can be withdrawn from methanol storage tank 140 to form third feed stream 107 which is introduced into methanol decomposition reactor 130 containing methanol decomposition catalyst 160, and second product stream 108 is withdrawn.

Embodiment 1. A process for the production of a H₂/CO stream comprising hydrogen and carbon monoxide, the process comprising:
for a first period of time,
   providing a first feed stream comprising hydrogen and CO₂;
   contacting the first feed stream with a hydrogenation catalyst (e.g., in a hydrogenation reactor) to form a first product stream comprising synthesized methanol;
   providing a second feed stream comprising at least a portion of the synthesized methanol of the first product stream;
   contacting the second feed stream with a methanol decomposition catalyst (e.g., in a methanol decomposition reactor) to decompose at least a portion of the methanol to form a second product stream comprising CO and H₂; and
   providing at least a portion of the CO and the H₂ of the second product stream to the H₂/CO stream; and
for a second period of time,
   providing a source of stored methanol;
   providing a third feed stream comprising stored methanol;
   contacting the third feed stream with the methanol decomposition catalyst (e.g., in a methanol decomposition reactor) to decompose at least a portion of the methanol to form a third product stream comprising CO and H₂; and
   providing at least a portion of the CO and the H2 of the third product stream to the H₂/CO stream,
wherein a synthesized methanol fraction of the second feed stream is substantially greater than a synthesized methanol fraction of the third feed stream.

Embodiment 2. The process of embodiment 1, wherein the hydrogen of the first feed stream comprises green hydrogen or blue hydrogen.

Embodiment 3. The process of embodiment 1 or embodiment 2, wherein the hydrogen of the first feed stream comprises green hydrogen.

Embodiment 4. The process of any of embodiments 1-3, wherein the process further comprises electrolyzing water to form an electrolysis product stream comprising hydrogen, and providing hydrogen from the electrolysis product stream to the first feed stream.

Embodiment 5. The process of embodiment 4, wherein the electrolysis is performed using electricity from a renewable source.

Embodiment 6. The process of embodiment 5, wherein the renewable source of electricity is provided by one or more of solar energy, wind energy, and hydroelectric energy (such as tidal energy).

Embodiment 7. The process of any of embodiments 4-6, wherein the hydrogen produced by electrolysis is not stored for a substantial amount of time (e.g., no more than one hour). Embodiment 8. The process of any of embodiments 4-7, wherein at least a portion of the hydrogen produced by electrolysis is provided to the H2/CO stream while bypassing the hydrogenation of CO₂.

Embodiment 9. The process of any of embodiments 1-8, wherein the first feed stream comprises at least 10 vol% H₂, e.g., at least 20 vol% H₂, or at least 30 vol% H₂.

Embodiment 10. The process of any of embodiments 1-9, wherein the first feed stream comprises at least 5 vol% CO₂, e.g., at least 10 vol% CO₂, or at least 15 vol% CO₂.

Embodiment 11. The process of any of embodiments 1-10, wherein the CO₂ of the first feed stream comprises captured CO₂ (e.g., direct air captured CO₂, or captured waste CO₂, such as biomass-derived CO₂), e.g., comprises at least 50%, at least 75%, at least 90% or at least 95% captured CO₂.

Embodiment 12. The process of any of embodiments 1-10, wherein the CO₂ of the first feed stream comprises carbon dioxide from biomass gasification, e.g., comprises at least 20%, at least 50%, at least 75%, at least 90% or at least 95% carbon dioxide from biomass gasification.

Embodiment 13. The process of any of embodiments 1-11, wherein the first feed stream further comprises methane.

Embodiment 14. The process of any of embodiments 1-11, wherein an amount of methane in the first feed stream is no more than 50 mol% of carbon-bearing species, e.g., no more than 30 mol%, or no more than 20 mol%, or no more than 10 mol%, or no more than 5 mol%. Embodiment1 5. The process of any of embodiments 1-14 wherein a ratio of H₂:CO₂ of the first feed stream is at least 1:1, e.g., at least 1.5:1, on a molar basis.

Embodiment 16. The process of any of embodiments 1-14, wherein a ratio of H₂:CO₂ of the first feed stream is at least 2:1, e.g., at least 2.5:1, or at least 3:1, or at least 4:1, or at least 5:1, or at least 6:1, or at least 8:1.

Embodiment 17. The process of any of embodiments 1-16, wherein a ratio of H₂:CO₂ of the first feed stream is no more than 25:1, e.g., no more than 20:1, or no more than 15:1, or no more than 12:1.

Embodiment 18. The process of any of embodiments 1-17, wherein the first feed stream comprises no more than 1% O₂, for example, no more than 0.1% O₂, or no more than 0.01% O₂, or substantially no O₂; and/or no more than 10 vol% CO (e.g., no more than 5 vol%, or 3 vol%, or 2 vol%, or 1 vol%, or 0.1 vol% CO).

Embodiment 19. The process of any embodiments 1-18, wherein the contacting of the first feed stream with the hydrogenation catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.

Embodiment 20. The process of any of embodiments 1-19, wherein the contacting of the first feed stream with the hydrogenation catalyst is performed at a pressure of no more than 100 bar, for example, no more than 80 bar, or no more than 60 bar.

Embodiment 21. The process according to any of embodiments 1-20, wherein the formation of the first product stream is performed with a selectivity of at least 50% for methanol, e.g., at least 55%, or at least 60%.

Embodiment 22. The process according to any of embodiments 1-20, wherein the formation of the first product stream is performed with a selectivity of at least 65% for methanol (e.g., at least 70%, or at least 75%, or at least 80% for methanol).

Embodiment 23. The process according to any of embodiments 1-20, wherein the formation of the first product stream is performed with a selectivity of no more than 20% for methane, e.g., no more than 10%, or no more than 5% for methane.

Embodiment 24. The process according to any of embodiments 1-23, wherein the formation of the first product stream is performed with a selectivity of no more than 20% for CO, e.g., no more than 10%, or no more than 5% for CO.

Embodiment 25. The process according to any of embodiments 1-23, wherein the formation of the first product stream is performed with a selectivity of no more than 2% for CO, or 1% for CO.

Embodiment 26..The process according to any of embodiments 1-25, wherein the formation of the first product stream is performed with a conversion of CO₂ of at least 25%, e.g. at least 35%, e.g., at least 45% or at least 50%.

Embodiment 27. The process according to any of embodiments 1-26, wherein the first product stream comprises at least 15 vol% methanol, e.g., at least 25 vol%, or at least 35 vol% methanol.

Embodiment 28. The process according to any of embodiments 1-27, wherein the first product stream comprises no more than 10 vol% methane, e.g., no more than 5 vol% methane, or no more than 2 vol% methane.

Embodiment 29. The process according to any of embodiments 1-28, wherein the first product stream includes no more than 10 vol% CO, e.g., no more than 5 vol% CO, or no more than 2 vol% CO.

Embodiment 30. The process according to any of embodiments 1-29, further comprising separating at least a portion of water of the first product stream therefrom, e.g., separating at least 50%, or at least 75%, or at least 90%, or at least 95%, or at least 99%, or at least 99.5% of the water of the first product stream.

Embodiment 31. The process according to any of embodiments 1-30, wherein the portion of the first product stream that is included in the second feed stream has a water content of no more than 10 mol%, e.g., no more than 2 mol%, or no more than 1 mol%, or no more than 0.5 mol%.

Embodiment 32. The process according to any of embodiments 1-31, further comprising separating at least a portion of H₂ from the first product stream, e.g., at least 50%, or at least 60%, or at least 75%, or at least 90%, or at least 95% of the H₂ in the first product stream. Embodiment 33. The process according to embodiment 32, wherein at least a portion of the separated H₂ is recycled to the first feed stream, and/or wherein at least a portion of the separated H₂ provided as a portion of a Fischer-Tropsch feed stream, and/or wherein at least a portion of the separated H₂ is used to activate a methanol decomposition catalyst and/or a Fischer-Tropsch catalyst.

Embodiment 34. The process according to any of embodiments 1-33, wherein the contacting of the second feed stream and/or third feed stream with the methanol decomposition catalyst and the contacting of the first feed stream with the hydrogenation catalyst are performed in different plants.

Embodiment 35. The process according to any of embodiments 1-34, wherein the first characteristic process time is in the range of 30 minutes to 4 hours, e.g., in the range of 30 minutes to 3.5 hours, e.g., 30 minutes to 3 hours, or 30 minutes to 2.5 hours, or 30 minutes to 2 hours, or 30 minutes to 1.5 hours, or 30 minutes to 1 hour.

Embodiment 36. The process according to any of embodiments 1-34, wherein the first characteristic process time is in the range of 1-4 hours, e.g., 1-3.5 hours, or 1-3 hours, or 1-2.5 hours, or 1-2 hours, or 1-1.5 hours.

Embodiment 37. The process according to any of embodiments 1-34, wherein the first characteristic process time is in the range of 1.5-4 hours, e.g., 1.5-3.5 hours, or 1.5-3 hours, or 1.5-2.5 hours, or 1.5-2 hours.

Embodiment 38. The process according to any of embodiments 1-34, wherein the first characteristic process time is in the range of 2-4 hours, e.g., 2-3.5 hours, or 2-3 hours, or 2-2.5 hours.

Embodiment 39. The process according to any of embodiments 1-34, wherein the first characteristic process time is in the range of 2.5-4 hours, e.g., 2.5-3.5 hours, or 2.5-3 hours.

Embodiment 40. The process according to any of embodiments 1-34, wherein the first characteristic process time is in the range of 3-4 hours, e.g., 3-3.5 hours, or 3.5-4 hours.

Embodiment 41. The process according to any of embodiments 1-34, wherein the first characteristic process time is 30 minutes.

Embodiment 42. The process according to any of embodiments 1-34, wherein the first characteristic process time is 1 hour.

Embodiment 43. The process according to any of embodiments 1-34, wherein the first characteristic process time is 1.5 hours.

Embodiment 44. The process according to any of embodiments 1-34, wherein the first characteristic process time is 2 hours.

Embodiment 45. The process according to any of embodiments 1-34, wherein the first characteristic process time is 2.5 hours.

Embodiment 46. The process according to any of embodiments 1-34, wherein the first characteristic process time is 3 hours.

Embodiment 47. The process according to any of embodiments 1-34, wherein the first characteristic process time is 3.5 hours.

Embodiment 48. The process according to any of embodiments 1-34, wherein the first characteristic process time is 4 hours.

Embodiment49. The process of any of embodiments 1-45, further comprising, during the first period of time, storing at least a portion of the methanol of the first product stream.

Embodiment 50. The process of embodiment 49, wherein methanol stored during the first period of time is provided to the third feed stream during the second period of time.

Embodiment 51. The process of any of embodiments 1-50, wherein substantially all of the methanol of the second feed stream is synthesized methanol.

Embodiment 52. The process of any of embodiments 1-50, wherein the second feed stream has a synthesized methanol fraction of at least 95%, e.g., at least 98%, or at least 99%.

Embodiment 53. The process of any of embodiments 1-50, wherein the second feed stream has a synthesized methanol fraction of at least 50%, e.g., at least 65%, or at least 75%.

Embodiment 54. The process of any of embodiments 1-50, wherein the second feed stream has a synthesized methanol fraction of at least 80%, e.g., at least 85%, or at least 90%.

Embodiment 55. The process of any of embodiments 1-54, wherein the third feed stream has a synthesized methanol fraction of no more than 90%, e.g., no more than 85%, or no more than 80%.

Embodiment 56. The process of any of embodiments 1-54, wherein the third feed stream has a synthesized methanol fraction of no more than 75%, e.g., no more than 70%, or no more than 65 wt%.

Embodiment 57. The process of any of embodiments 1-54, wherein the third feed stream has a synthesized methanol fraction of no more than 60 wt%, e.g., no more than 55 wt%, or no more than 50 wt%.

Embodiment 58. The process of any of embodiments 1-54, wherein the third feed stream has a synthesized methanol fraction of no more than 45%, e.g., no more than 40%, or no more than 35%.

Embodiment 59. The process of any of embodiments 1-54, wherein the third feed stream has a synthesized methanol fraction of no more than 30%, e.g., no more than 25%, or no more than 20%.

Embodiment 60. The process of any of embodiments 1-54, wherein the third feed stream has a synthesized methanol fraction of no more than 15%, e.g., no more than 10%, or no more than 5%.

Embodiment 61. The process of any of embodiments 1-60, wherein substantially all of the methanol of the third feed stream is stored methanol.

Embodiment 62. The process of any of embodiments 1-60, wherein the third feed stream has a stored methanol fraction of at least 5%, e.g., at least 10%, or at least 15%.

Embodiment 63. The process of any of embodiments 1-60, wherein the third feed stream has a stored methanol fraction of at least 20%, e.g., at least 25%, or at least 30%.

Embodiment 64. The process of any of embodiments 1-60, wherein the third feed stream has a stored methanol fraction of at least 35%, e.g., at least 40%, or at least 45%.

Embodiment 65. The process of any of embodiments 1-60, wherein the third feed stream has a stored methanol fraction of at least 50%, e.g., at least 55%, or at least 60%.

Embodiment 66. The process of any of embodiments 1-60, wherein the third feed stream has a stored methanol fraction of at least 65%, e.g., at least 70%, or at least 75%.

Embodiment 67. The process of any of embodiments 1-60, wherein the third feed stream has a stored methanol fraction of at least 80%, e.g., at least 85%, or at least 90%.

Embodiment 68. The process of any of embodiments 1-60, wherein the third feed stream has a stored methanol fraction of at least 95%, e.g., at least 98%, or at least 99%.

Embodiment 69. The process of any of embodiments 61-68, wherein stored methanol of the third feed stream has a storage time of at least three hours, e.g., at least five hours, or at least eight hours.

Embodiment 70. The process of any of embodiments 61-68, wherein stored methanol of the third feed stream has a storage time of at least twelve hours, e.g., at least 18 hours, or at least 24 hours.

Embodiment 71. The process of any of embodiments 61-68, wherein stored methanol of the third feed stream has a storage time of at least thirty-six hours, e.g., at least three days or at least seven days.

Embodiment 72. The process of any of embodiments 61-68, wherein stored methanol of the third feed stream has a storage time of no more than 30 days, e.g., no more than 21 days, or no more than 14 days, or no more than seven days.

Embodiment 73. The process of any of embodiments 61-68, wherein stored methanol of the third feed stream has a storage time of no more than three days, e.g., no more than two days, or no more than one day.

Embodiment 74. The process of any of embodiments 1-73, wherein a difference between the synthesized methanol fraction of the second feed stream and the synthesized methanol fraction of the third feed stream is at least 5%, e.g., at least 10%, or at least 15%.

Embodiment 75. The process of any of embodiments 1-73, wherein a difference between the synthesized methanol fraction of the second feed stream and the synthesized methanol fraction of the third feed stream is at least 20%, e.g., at least 25%, or at least 30%.

Embodiment 76. The process of any of embodiments 1-73, wherein a difference between the synthesized methanol fraction of the second feed stream and the synthesized methanol fraction of the third feed stream is at least 35%, e.g., at least 40%, or at least 45%.

Embodiment 77. The process of any of embodiments 1-73, wherein a difference between the synthesized methanol fraction of the second feed stream and the synthesized methanol fraction of the third feed stream is at least 50%, e.g., at least 55%, or at least 60%.

Embodiment 78. The process of any of embodiments 1-73, wherein a difference between the synthesized methanol fraction of the second feed stream and the synthesized methanol fraction of the third feed stream is at least 65%, e.g., at least 70%, or at least 75%.

Embodiment 79. The process of any of embodiments 1-78, wherein the second period of time is in the range of 30 minutes to 24 hours in length (e.g., 30 minutes to 18 hours, or 30 minutes to 12 hours, or 1-24 hours, or 1-18 hours, or 1-12 hours, or 3-24 hours, or 3-18 hours, or 3-12 hours, or 6-24 hours, or 6-18 hours, or 6-12 hours), and occurs after the first period of time.

Embodiment 80. The process of any of embodiments 1-79, wherein the second period of time is followed by another first period of time.

Embodiment 81. The process of any of embodiments 1-79, wherein the process includes a plurality of alternating first and second periods of time.

Embodiment 82. The process of any of embodiments 1-81, further comprising including at least a portion of the second product stream to the first feed stream.

Embodiment 83. The process of any of embodiments 1-82, wherein the H₂/CO stream further comprises at least a portion of hydrogen not derived from methanol decomposition.

Embodiment 84. The process of any of embodiments 1-83, wherein the first feed stream comprises no more than 10 vol% CO.

Embodiment 85. The process of any of embodiments 1-84, wherein the H₂/CO stream further comprises at least a portion of carbon monoxide not derived from methanol decomposition.

Embodiment 86. The process of any of embodiments 1-85, further comprising contacting at least a portion of the H₂/CO stream with the hydrogenation catalyst (e.g., in the hydrogenation reactor).

Embodiment 87. A process for the production of hydrocarbons, the process comprising performing a Fischer-Tropsch reaction by contacting at least a portion of the H₂/CO stream of any of embodiments 1-86 with a Fischer-Tropsch catalyst (e.g., in a Fischer-Tropsch reactor) to provide a Fischer-Tropsch product stream comprising hydrocarbons, water, and optionally oxygenated hydrocarbons.

Embodiment 88. The process of embodiment 87, wherein the Fischer-Tropsch reaction has a desired throughput, and wherein during the first period of time, a supply of electricity from a renewable source is sufficient to provide the desired throughput, and wherein during the second period of time, the supply of electricity from a renewable source is insufficient to provide the desired throughput.

Embodiment 89. The process of embodiments 87 or embodiment 88, wherein the Fischer-Tropsch product stream comprises carbon dioxide, wherein the process further comprises recycling at least a portion of the carbon dioxide to the first feed stream.

The particulars shown herein are by way of example and for purposes of illustrative discussion of various embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words `comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for the production of a H₂/CO stream comprising hydrogen and carbon monoxide, the process comprising:
for a first period of time,
providing a first feed stream comprising hydrogen and CO₂;
contacting the first feed stream with a hydrogenation catalyst (e.g., in a hydrogenation reactor) to form a first product stream comprising synthesized methanol;
providing a second feed stream comprising at least a portion of the synthesized methanol of the first product stream;
contacting the second feed stream with a methanol decomposition catalyst (e.g., in a methanol decomposition reactor) to decompose at least a portion of the methanol to form a second product stream comprising CO and H₂; and
providing at least a portion of the CO and the H₂ of the second product stream to the H₂/CO stream; and
for a second period of time,
providing a source of stored methanol;
providing a third feed stream comprising stored methanol;
contacting the third feed stream with the methanol decomposition catalyst (e.g., in a methanol decomposition reactor) to decompose at least a portion of the methanol to form a third product stream comprising CO and H₂; and
providing at least a portion of the CO and the H2 of the third product stream to the H₂/CO stream,
wherein a synthesized methanol fraction of the second feed stream is substantially greater than a synthesized methanol fraction of the third feed stream.

2. The process of claim 1, wherein the process further comprises electrolyzing water to form an electrolysis product stream comprising hydrogen, and providing hydrogen from the electrolysis product stream to the first feed stream, wherein the electrolysis is performed using electricity from a renewable source.

3. The process according to claim 1 or claim 2, wherein the formation of the first product stream is performed with a selectivity of at least 65% for methanol.

4. The process according to any of claims 1-3, wherein the portion of the first product stream that is included in the second feed stream has a water content of no more than 2 mol%.

5. The process according to any of claims 1-4, further comprising separating at least a portion of H₂ from the first product stream, wherein at least a portion of the separated H₂ is recycled to the first feed stream, and/or wherein at least a portion of the separated H₂ provided as a portion of a Fischer-Tropsch feed stream, and/or wherein at least a portion of the separated H₂ is used to activate a methanol decomposition catalyst and/or a Fischer-Tropsch catalyst.

6. The process according to any of claims 1-5, wherein the first characteristic process time is in the range of 30 minutes to 4 hours.

7. The process according to any of claims 1-6, wherein the first characteristic process time is 30 minutes, or is 2 hours.

8. The process of any of claims 1-7, wherein the second feed stream has a synthesized methanol fraction of at least 95%.

9. The process of any of claims 1-8, wherein the third feed stream has a synthesized methanol fraction of no more than 70%.

10. The process of any of claims 1-9, wherein the third feed stream has a stored methanol fraction of at least 20%.

11. The process of claim 10, wherein stored methanol of the third feed stream has a storage time of at least three hours.

12. The process of any of claims 1-11, wherein a difference between the synthesized methanol fraction of the second feed stream and the synthesized methanol fraction of the third feed stream is at least 20%.

13. The process of any of claims 1-12, wherein the process includes a plurality of alternating first and second periods of time.

14. The process of any of claims 1-13, wherein the H₂/CO stream further comprises at least a portion of hydrogen not derived from methanol decomposition.

15. A process for the production of hydrocarbons, the process comprising performing a Fischer-Tropsch reaction by contacting at least a portion of the H₂/CO stream prepared by the process of any of claims 1-14 with a Fischer-Tropsch catalyst (e.g., in a Fischer-Tropsch reactor) to provide a Fischer-Tropsch product stream comprising hydrocarbons, water, and optionally oxygenated hydrocarbons.
